# EUROPEAN PATENT APPLICATION

(11) **EP 3 545 939 A1**
(43) Date of publication of application: **02.10.2019**
(21) Application number: 19161654.9
(22) Date of filing: 08.03.2019
(51) Int. Cl.: A61K 6/027, A61K 6/02, A61K 8/20

(54) **DENTAL MODELLING FLUIDS**

(30) Priority: 08.03.2018 GB 201803722
(71) Applicant: Zhubi, Fatos Riza, London, Greater London WC1X 0BA (GB); Jahja, Kemal Hamdi, 1000 Prishtina (RS)
(72) Inventor: Zhubi, Fatos Riza, London, Greater London WC1X 0BA (GB); Jahja, Kemal Hamdi, 1000 Prishtina (RS)
(74) Representative: Boult Wade Tennant LLP

(57) **Abstract**

The present invention relates to a composition for producing a dental paste, wherein the composition comprises (i) 0.01 to 0.13 w/v% NaCl and (ii) 0.01 to 1.04 w/v% KCI in water. The present invention also relates to a dental paste comprising the composition and a dental powder. The present invention further relates to use of the composition or dental paste for producing a dental prosthesis or an opaquer.

## Description

### FIELD OF THE INVENTION

The present invention relates to a composition for producing a dental paste. The composition comprises sodium chloride (NaCI) and potassium chloride (KCI) in water, preferably distilled water. The composition may be combined with a dental modelling powder or an opaque powder to produce a dental paste. The present invention provides uses of the composition and uses of the dental paste comprising the composition for producing dental restoratives, including producing dental prostheses. The composition and dental paste of the present invention can also be used to produce opaquers. The dental restoratives may include crowns, bridges, pontics, inlays, onlays, dentures, laminates, and veneers. The present invention also provides a process for manufacturing a dental paste, and a process for manufacturing a dental prosthesis.

### BACKGROUND TO THE INVENTION

A dental restoration is a treatment to restore the function, integrity, and morphology of missing tooth structure resulting from caries or external trauma. An indirect restoration may be fabricated outside of the mouth by a dental technician using dental impressions of the prepared tooth provided by the dentist. Common indirect restorations include crowns, bridges, pontics, inlays, onlays, dentures, laminates, and veneers. Common direct restorations include filings.

Various materials are used by dental technicians when producing or correcting a dental restorative. Typically a powder is mixed with a liquid to produce a dental paste with the desired consistency such that it can be moulded and carved into shape. This moulded prosthesis can then be fixed onto either (i) a prepared tooth, in situations where some of the original tooth remains intact, or may be (ii) fixed onto a dental implant, in situations where the original tooth has had to be replaced entirely by a substructure.

When manufacturing the prosthesis, it is important that the dental paste is stable over a sufficient period of time such that the dental technician can carve and contour the prosthesis during the modelling phase, without the paste drying too quickly and producing unwanted cracks. The materials used to produce the prosthesis must also be able to endure the baking phase, when the moulded prosthesis is heated in a furnace. It is important that the prosthesis is able to withstand high temperatures, without cracking, and whilst preserving the desired colour, shade and luminescence required of an artificial tooth.

A dental substructure, such as a dental implant, is typically made of metal and therefore the dental technician must first apply a layer of opaque ceramic paste. This layer ensures a good bond to the metal implant and to the veneering prosthesis, and in addition masks the unfavourable colour of the metalwork to provide a colour that aesthetically matches the surrounding teeth. The prosthesis is then applied over the opaque layer, and is usually bonded permanently with a dental cement.

It is preferable that the final dental restorative remain intact for as long as possible. Ideally the prosthesis will remain fixed to the dental implant, and will retain its strength and stability such that it does not crack or chip. It is also desirable that the colour of the artificial tooth is preserved over time.

Typical dental pastes used to construct the prosthesis may comprise powders of different types of ceramics, porcelains, and zirconia, depending on the type of restoration and the location of the tooth to be restored. Water has been used as a standard binding agent for dental powders, such as porcelain powders. However, one of the disadvantages of using water is that water evaporates too quickly from the powder-water mix, making the porcelain prosthesis too fragile for the technician to handle, resulting in the prosthesis becoming chipped or cracked. Attempts to add more water to the mix can result in a composition that has poor adhesive properties making it difficult to apply to most surfaces. Technicians have attempted to overcome some of the disadvantages associated with using water. For example, technicians have tried substituting some or all of the water with lower alkyl polyhydric alcohols and ethers of alcohols (US3880662), or with special modelling liquids, such as a polymerizable resin modelling fluid. Typical resins may be one-component or multi-component resins and may include a polyester monophosphate, a polyether monophosphate, or a polyurethane monophosphate, for example (EP0328772).

Despite a number of available modelling liquids there exists a need to provide improved compositions that can be mixed with dental powders to produce novel dental pastes that retain the desired properties both during handling, baking and thereafter.

### SUMMARY OF THE INVENTION

The present invention relates to a dental composition having improved properties. The composition can be used to produce a dental paste which is particularly effective for producing dental prostheses and opaquers. The composition is water-based and comprises salts in order to reduce the amount and rate of water evaporation from the paste during modelling.

In a first aspect, the present invention provides a composition for producing a dental paste, wherein the composition comprises: (i) 0.01 to 0.13 w/v % NaCl and (ii) 0.01 to 1.04 w/v % KCI in water. Preferably, the water is distilled water.

In a second aspect, the present invention provides a dental paste comprising (i) 0.01 to 0.13 w/v % NaCl and 0.01 to 1.04 w/v % KCI in water, and (ii) a dental powder. Preferably, the water is distilled water.

The dental powder may be selected from: a ceramic powder, a porcelain powder, an opaque powder, an enamel powder, a zirconium powder, and combinations thereof.

In certain embodiments, the dental paste may further comprise additional agents, for example a stabiliser and/or a preservative.

The composition and dental paste of the invention may be used for producing a dental prosthesis, such as a crown, bridge, pontic, inlay, onlay, denture, laminate, or veneer. The composition and dental paste may be used for correcting an existing dental prosthesis. In addition, the composition and dental paste of the invention may be used for producing an opaquer.

In another aspect, the invention provides a process for manufacturing a dental paste wherein the process comprises mixing the composition according to the invention with a dental powder. Accordingly, the invention provides a dental paste obtainable by the same process.

The invention further provides a process for manufacturing a dental prosthesis, wherein the process comprises: a) mixing the composition according to the invention with a dental powder to produce a dental paste; b) modelling the paste into a dental prosthesis; c) polishing and/or trimming the dental prosthesis; and d) baking the dental prosthesis, optionally with a dental glaze.

The invention further provides a kit comprising (i) a composition in accordance with invention, and (ii) a dental powder.

### BRIEF DESCRIPTION OF THE DRAWINGS

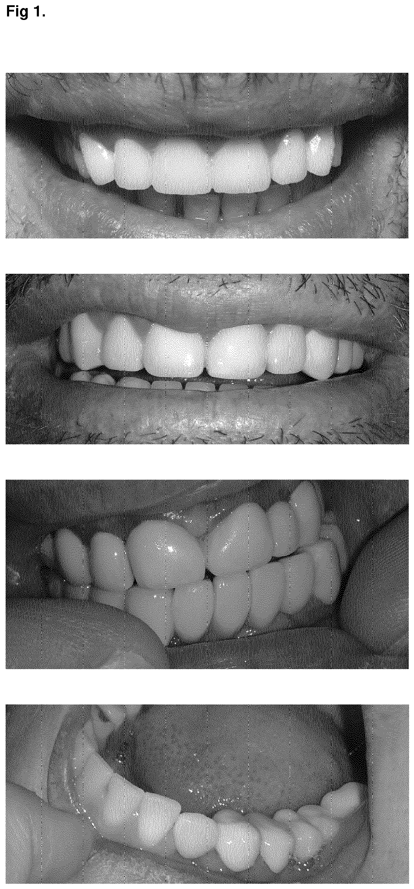

**FIGURE 1** shows pictures of successful dental restorations in accordance with the invention.

### DETAILED DESCRIPTION

### A. Definitions

Unless defined otherwise, all technical and scientific terms used herein have the same meaning as is commonly understood by one skilled in the art in the technical field of the invention.
**"Dental restorative"** - As used herein, the term "dental restorative" can be used interchangeably with "dental restoration", and is a broad term used to refer to a dental appliance used to preserve the function and/or aesthetics of natural teeth. The term may be used to refer to the whole dental appliance including both the underlying metalwork, for example a dental implant, and the overlaid dental prosthesis. Non-limiting examples of dental restoratives include crowns, bridges, pontics, inlays, onlays, full and partial dentures, laminates, veneers, and dental implants. A dental restorative may be used to replace missing teeth and to help prevent cavities in the remaining teeth since empty spaces caused by missing teeth are vulnerable spots for plaque-causing bacteria to build up. A dental restorative generally refers to an indirect restoration. Indirect tooth restoration involves customized tooth replacements which are fabricated by a dental technician outside of the mouth using dental impressions of the prepared tooth. Non-limiting examples of indirect restoratives include crowns, bridges, pontics, inlays, onlays, full and partial dentures, laminates, and veneers.
**"Dental prosthesis"** - As used herein, the terms "dental prosthesis", "artificial prosthesis", "moulded prosthesis" or "prosthesis" are used to refer to the customized tooth replacements which may be overlaid on a dental implant, or over an existing prepared tooth. Like other types of prostheses, they can either be fixed permanently or removable. The term is intended to encompass part of the dental restorative that is modelled and shaped by the dental technician, and which forms the whole or part of the external part of an artificial tooth. The prosthesis may, for example, be made of cast metal, such as gold; ceramics, such as porcelain; composite materials; ceramometals; or zirconia. Non-limiting examples of dental prostheses include: crowns, bridges, pontics, inlays, onlays, full and partial dentures, laminates, and veneers.
**"Crown"** - As used herein, the term "crown" refers to a dental restorative used to entirely cover or "cap" a damaged tooth. Besides strengthening a damaged tooth, a crown can be used to improve its appearance, shape or alignment. A crown can also be placed on top of a dental implant to provide a tooth-like shape and structure for function. Porcelain or ceramic crowns can be matched to the colour of the surrounding natural teeth. Other materials include gold and metal alloys, and acrylic. These alloys are generally stronger than porcelain and may be recommended for molars. Porcelain bonded to a metal shell is often used because it is both strong and attractive.
**"Bridge"** - As used herein, the term "bridge" refers to a dental restorative recommended if a subject is missing one or more teeth. Gaps left by missing teeth eventually cause the remaining teeth to rotate or shift into the empty spaces, resulting in a bad bite. The imbalance caused by missing teeth can also lead to gum disease and temporomandibular joint (TMJ) disorders. Bridges are commonly used to replace one or more missing teeth. They span the space where the teeth are missing. Bridges are cemented to the natural teeth or implants surrounding the empty space. These teeth, called abutments, serve as anchors for the bridge.
**"Dental powder"** - As used herein, the term "dental powder" refers to any powder that may be mixed with a composition of the present invention in order to produce a dental paste, such as a modelling paste or an opaque paste. The powder may be a ceramic or porcelain powder. The powder may comprise silica (SiO₂), alumina (Al₂O₃), leucite, lithium disilicate, sodium aluminium silicate (soda feldspar), potassium aluminium silicate (potash feldspar), zirconia, zirconium oxide, or combinations thereof. The powder may be a zirconium powder. The powder may be an opaque powder. The opaque powder may comprise titanium oxide (TiO₂), aluminium oxide (Al₂O₃), and/or zirconium oxide (ZrO₂). The powder may be a commercially available powder known in the art. For example, the dental powder may be purchased from BAOT®, Ivoclar Vivident®, or Vita.
**"Modelling fluid"** - As used herein, the term "modelling fluid" is used to refer to a composition which when mixed with a dental powder will form a dental paste, such as a modelling paste or an opaque paste. The modelling fluid may act as a binding agent to bind the powder particles together and ensure the required consistency of the resultant dental paste. The term includes known modelling fluids such as water, alcohols, and resins. The modelling fluid may be a commercially available composition known in the art. Commercially available modelling fluids may be purchased, for example, from BAOT®, Ivoclar Vivident®, or Vita. The modelling fluid of the present invention is a composition comprising a specific quantity of NaCl and KCI in water. The modelling fluid may be in liquid form. The modelling fluid may be an aqueous solution. The modelling fluid may be a solvent.
**"Dental paste"** - As used herein, the term "dental paste" is used to encompass both modelling pastes and opaque pastes, which are further explained below.
**"Modelling paste"** - As used herein, the term "modelling paste" is used to refer to the paste that is moulded and/or carved by a dental technician in order to produce a dental prosthesis. The modelling paste may be formed from a mixture of a modelling powder, such as a ceramic or porcelain powder, with a modelling fluid.
**"Opaquer"** - As used herein, the term "opaquer" is used interchangeably with "opaque paste" and is used to describe a product that masks the dark colour and shades of the underlying metallic framework of a dental restorative. The opaquer may also be used to mask tooth discolouration. The opaquer may be formed from a mixture of an opaque powder and a modelling fluid. The opaquer may be formed from a composite resin material.

### B. Composition

The present invention relates to a composition for producing a dental paste. The present inventors have discovered a composition that acts as a novel modelling fluid with improved properties.

In a first aspect, the present invention provides a composition for producing a dental paste, wherein the composition comprises: (i) 0.01 to 0.13 w/v % NaCl and (ii) 0.01 to 1.04 w/v % KCI in water. In a further embodiment, the present invention provides a composition for producing a dental paste, wherein the composition comprises: (i) 0.05 to 0.13 w/v % NaCl and (ii) 0.05 to 1.04 w/v % KCI in water. In certain embodiments, the present invention provides a composition for producing a dental paste, wherein the composition comprises: (i) 0.08 to 0.13 w/v % NaCl and (ii) 0.08 to 1.04 w/v % KCI in water. In preferred embodiments, the present invention provides a composition for producing a dental paste, wherein the composition comprises: (i) 0.1 w/v % NaCl and (ii) 0.6 w/v % KCI in water. In an alternative embodiment, the present invention provides a composition for producing a dental paste, wherein the composition comprises: (i) 0.09 w/v % NaCl and (ii) 0.06 w/v % KCI in water.

In an alternative embodiment, the present invention provides a dental fluid for producing a dental paste, wherein the dental fluid comprises: (i) 0.01 to 0.13 w/v % NaCl and (ii) 0.01 to 1.04 w/v % KCI in water; (i) 0.05 to 0.13 w/v % NaCl and (ii) 0.05 to 1.04 w/v % KCI in water; (i) 0.08 to 0.13 w/v % NaCl and (ii) 0.08 to 1.04 w/v % KCI in water; (i) 0.1 w/v % NaCl and (ii) 0.6 w/v % KCI in water; or i) 0.09 w/v % NaCl and (ii) 0.60 w/v % KCI in water.

In each case, the percentage concentration is calculated as the fraction of the weight of the solute related to the total volume of the solution. The amount (weight) of the NaCl and KCI is expressed as a percentage of the total solution volume. The percentages are calculated in the form of weight/volume % (wt/vol % or w/v %).

In certain embodiments, the composition of the present invention comprises distilled water. Distilled water may be used to reduce the chance of contamination that may adversely affect the colour or shade of the resultant dental paste. Advantageously, since the composition comprises only natural ingredients, the composition has no associated health risks, and is therefore safe for use in humans and animals.

The present inventors have shown that a composition comprising specific quantities of both NaCl and KCI in water possesses a number of improved properties. The composition can be combined with a dental powder in order to produce a dental paste with improved properties. Without wishing to be bound by theory it is understood that a composition comprising water alone does not produce a suitable dental paste because the water evaporates too quickly from the powder-water mix, resulting in the paste drying out too quickly. This does not leave enough time for the dental technician to handle the paste, either to model the dental paste into a prosthesis, or to paint further layers of the dental paste onto a prosthesis to build up the desired shape. As the water evaporates, the dental paste will dry out and is more prone to chipping and/or cracking. The present inventors have discovered that addition of both NaCl and KCI to the water results in a composition wherein the water evaporates more slowly from the powder-water mix. The dental paste is therefore more stable, less prone to chipping and/or cracking, and easier to handle and manipulate. Specifically, the present inventors have found that addition of up to about 0.13 w/v % NaCl, and addition of up to about 1.04 w/v % KCI to the water is particularly advantageous.

### C. Dental paste

In a second aspect, the present invention provides a dental paste comprising (i) a composition in accordance with the invention, and (ii) a dental powder. In a further embodiment, the present invention provides a dental paste comprising (i) 0.01 to 0.13 w/v % NaCl and 0.01 to 1.04 w/v % KCI in water, and (ii) a dental powder. In another embodiment, the present invention provides a dental paste comprising (i) 0.05 to 0.13 w/v % NaCl and 0.05 to 1.04 w/v % KCI in water, and (ii) a dental powder. In certain embodiments, the present invention provides a dental paste comprising (i) 0.08 to 0.13 w/v % NaCl and 0.08 to 1.04 w/v % KCI in water, and (ii) a dental powder. In preferred embodiments, the present invention provides a dental paste comprising (i) 0.1 w/v % NaCl and 0.6 w/v % KCI in water, and (ii) a dental powder. In preferred embodiments, the present invention provides a dental paste comprising (i) 0.09 w/v % NaCl and 0.60 w/v % KCI in water, and (ii) a dental powder.

The present inventors have shown that a composition in accordance with the invention can be mixed with a dental powder to produce a dental paste having improved properties. Importantly, the present inventors have shown that the composition may be mixed with any type of dental powder and the resultant dental paste will possess the improved properties. A particularly advantageous property of the composition of the present invention is that the composition can be mixed with modelling powders to produce modelling pastes, and the composition can be mixed with opaque powders to produce opaquers. This is a considerable improvement because the composition is suitable for multiple functions. In contrast, currently available modelling fluids, such as water, can only be used to produce modelling pastes, but are not suitable for use in producing opaquers.

In certain embodiments, the dental powder is selected from a ceramic powder, a porcelain powder, an opaque powder, an enamel powder, a zirconium powder, and combinations thereof. In certain embodiments, the dental powder comprises silica (SiO₂), alumina (Al₂O₃), leucite, lithium disilicate, sodium aluminium silicate (soda feldspar), potassium aluminium silicate (potash feldspar), zirconia, zirconium oxide, or combinations thereof. In alternate embodiments, the opaque powder comprises titanium oxide (TiO₂), aluminium oxide (Al₂O₃), zirconium oxide (ZrO₂), or combinations thereof.

The composition in accordance with the invention and the dental powder may be mixed together in any ratio required to produce a dental paste having the desired consistency. A dental technician may vary the amount of the composition and/or the amount of the powder. In certain embodiments, the composition forms 5-40 w/w % of the dental paste. In further embodiments, the composition forms 10-20 w/w % of the dental paste. In further embodiments, the composition forms about 25 w/w % of the dental paste. In preferred embodiments, the dental paste comprises a composition in accordance with the invention and a dental powder in a weight ratio of 1:3. In alternative embodiments, the ratio of composition to dental powder is 1:2. In alternative embodiments, the ratio of composition to dental powder is 1:4.

The dental paste of the present invention may comprise a single type of powder, or may comprise more than one type of powder. For example, the dental paste may comprise a combination of different types of ceramic powder, or a combination of a porcelain powder and a zirconium powder. Accordingly, in certain embodiments, the dental paste in accordance with the present invention may comprise one or more dental powders.

Additional agents may be included in the dental paste of the present invention. For example, the dental paste may comprise agents that increase stability or shelf-life. The dental paste may further comprise an agent that acts as a preservative. In certain embodiments, the dental paste according to the invention further comprises a stabiliser and/or a preservative.

The present inventors have discovered that the dental paste in accordance with the invention possesses improved properties. As explained elsewhere herein, the dental paste is not prone to drying out, and so is less likely to chip or crack during handling. In certain embodiments, the dental paste according to the invention is prevented from drying, chipping, and/or cracking. The dental paste may be prevented from drying, chipping, or cracking at any stage of the manufacturing process. In particular embodiments, the dental paste according to the invention is prevented from drying, chipping, and/or cracking during the modelling phase. In particular embodiments, the dental paste according to the invention is prevented from drying, chipping, and/or cracking during the baking phase. In particular embodiments, the composition in accordance with the invention evaporates from the dental paste gradually. Gradual evaporation of the composition may be assessed at room temperature. In certain embodiments, the dental paste stabilises about 30 minutes after the modelling phase. In certain embodiments, the dental paste retains its stability during the modelling phase and/or during the baking phase. The improved stability of the dental paste for a longer period of time means that the dental technician has more build-up control during the modelling phase.

The consistency of the dental paste is important for a number of reasons. For instance, the consistency will determine the final finish of the dental prosthesis. It is desirable that the surface of the prosthesis is smooth. In certain embodiments, the dental paste according to the invention is free of granules. In certain embodiments, the dental paste forms a homogeneous mixture. In addition, the correct consistency of the dental paste will make the paste easier to manipulate and model. It is preferable that the dental paste has a lasting softness and plasticity which will make carving and contouring easier without the need to re-soften the mixture. The viscosity of the dental paste is also important since this will determine the behaviour of the paste during handling. Ideally the viscosity of the dental paste is sufficient to allow thin layers of the paste to be applied. The viscosity of the dental paste can be measured by any method known in the art, for example by using a viscometer.

It is important that the dental paste is of the correct colour, shade and/or luminescence. A dental prosthesis is typically designed to match the colour, shade and/or luminescence of the surrounding natural teeth since this is more aesthetic. Similarly, an opaque paste is designed such that the colour, shade, and luminescence of the paste is suitable to mask the darker colours of the underlying metalwork or substructures. Consequently, it is important that the colour, shade, and luminescence of the dental powder does not change over time, or does not change under different conditions, such as during the baking phase. In preferred embodiments, the colour, shade and/or luminescence of the dental paste is preserved during and after baking. In certain embodiments, the dental paste can be baked without effecting discolouration. Colour, shade, and/or luminescence of the dental paste may be assessed by comparison with porcelain colour keys.

The consistency and properties of the dental paste are important in determining the quality of the overall finished product, whether that be a dental prosthetic or an opaquer. It is preferable that the final product is durable over an extended period of time. The prosthetic must be robust enough such that it is able to withstand daily chewing without chipping, cracking, or being displaced. In preferred embodiments, the dental paste in accordance with the invention retains its integrity for about 10 years, for about 8 years, for about 6 years, for about 4 years, for about 2 years, or for about 1 year. In preferred embodiments, the dental paste in accordance with the invention retains its stability for about 10 years, for about 8 years, for about 6 years, for about 4 years, for about 2 years, or for about 1 year. In preferred embodiments, the colour, shade and/or luminescence of the dental paste is preserved for about 10 years, for about 8 years, for about 6 years, for about 4 years, for about 2 years, or for about 1 year.

Accordingly, the dental paste may be required to adhere to the underlying dental substructures, such as metal implants. The dental paste may also be able to adhere to an opaque layer. In certain embodiments, the dental paste is able to adhere to a dental substructure. In certain embodiments, the dental paste is able to adhere to a metal implant. In certain embodiments, the dental paste is able to adhere to an opaquer.

### D. Uses

The composition and dental paste as described herein may be used for producing a dental prosthesis. Preferably, the composition and dental paste as described herein are for producing a dental prosthesis for a human subject.

In a further aspect, the present invention provides use of a composition in accordance with the invention, or a dental paste in accordance with the invention for producing a dental prosthesis. As described elsewhere herein, the dental prosthesis may be selected from: crowns, bridges, pontics, inlays, overlays, dentures, laminates, and veneers. In certain embodiments, the composition and dental paste according to the invention are for producing crowns. In certain embodiments, the composition and dental paste according to the invention are for producing bridges. In certain embodiments, the composition and dental paste according to the invention are for building veneers and/or laminates. In certain embodiments, the composition and dental paste according to the invention are for preparing inlays and/or onlays.

In further embodiments, the composition and dental paste according to the invention are for correcting a dental restorative. In particular embodiments, the composition and dental paste as described herein may be used to correct an existing prosthesis. In particular embodiments, the composition and dental paste according to the invention are for correcting a dental prosthesis.

In particular embodiments, the composition and dental paste according to the invention are for patching enamel. In particular embodiments, the composition and dental paste according to the invention are for connecting bridgework units.

In certain embodiments, the composition and dental paste as described herein may be used for producing an opaquer. Typically, when applying an opaque layer to a dental implant, the process requires a prior step whereby the metal implant is sandblasted to smooth any rough surfaces. Advantageously, the inventors have found that use of the dental paste as described herein allows for the production of an opaquer wherein the use of a sandblaster is not required. In certain embodiments, the composition and dental paste in accordance with the present invention are for use in producing an opaquer wherein the use of a sandblaster is eliminated. In certain embodiments, the composition and dental paste in accordance with the present invention are for use in producing a dental prosthesis wherein the use of a sandblaster is eliminated. In certain embodiments, the composition and dental paste in accordance with the present invention are for use in producing a dental prosthesis wherein the use of a sandblaster is eliminated prior to the opaque phase.

Other uses of the composition and dental paste described herein will be apparent to those skilled in the art.

### E. Process

In a further aspect, the present invention provides a number of manufacturing processes. The present invention provides a process for manufacturing a dental paste. In certain embodiments, the present invention provides a process for manufacturing a dental paste, wherein the process comprises mixing the composition in accordance with the invention with a dental powder. For example, the process may be for manufacturing a modelling paste, or for manufacturing an opaque paste.

In certain embodiments, the composition in accordance with the invention is mixed with a ceramic powder, a porcelain powder, an opaque powder, an enamel powder, a zirconium powder, or any combination thereof. The dental powder may comprise silica (SiO₂), alumina (Al₂O₃), leucite, lithium disilicate, sodium aluminium silicate (soda feldspar), potassium aluminium silicate (potash feldspar), zirconia, zirconium oxide, or any combination thereof. The dental powder may be a composite material. In certain embodiments, the opaque powder may comprise one or more opacifiers. In preferred embodiments, the opaque powder may comprise titanium oxide (TiO₂), aluminium oxide (Al₂O₃), zirconium oxide (ZrO₂), or any combination thereof.

To manufacture a dental paste, the composition in accordance with the present invention and the dental powder may be mixed in a weight ratio of 1:3. The dental technician may vary the amount of composition and/or the amount of dental powder in order to achieve the desired consistency. In certain embodiments, the composition and the dental powder may be mixed in a weight ratio of 1:2. In certain embodiments, the composition and the dental powder may be mixed in a weight ratio of 1:4. The composition and the dental powder may be mixed together at room temperature until the mixture reaches a homogenous consistency and is free of granules. In particular embodiments, the process for manufacturing a dental paste in accordance with the present invention involves mixing the composition and the dental powder at room temperature. In another embodiment, the process for manufacturing a dental paste in accordance with the present invention involves mixing the composition and the dental powder for at least 15 seconds, at least 25 seconds, at least 60 seconds, or at least 120 seconds. In particular embodiments, the process for manufacturing a dental paste in accordance with the present invention involves mixing the composition and the dental powder until the mixture is free of granules.

The present invention further provides a dental paste obtainable by the process described herein.

The present invention provides a process for manufacturing a dental prosthesis.

Prior to manufacturing the dental prosthesis, the underlying dental substructure, such as a dental implant, needs to be prepared. The dentist will typically use a paste or putty to make an impression of the tooth that is to receive the dental implant, such as those used to prepare a crown or a bridge. Impressions of the teeth above and below the tooth to receive the dental implant may also be made to make sure that the dental implant will not affect the subject's bite. The impressions are sent to a dental technician where the dental implant will be manufactured. A composition comprising gypsum and water is prepared and the mixture poured in the impression. Once the gypsum composition has hardened, it is removed from the impression, cleaned and then waxed. The waxed mould will then be converted into a replicate formed from a metal alloy. The metal will be processed, for example, using a micromotor. Typically, the metal may be polished using a sandblaster. However, the present inventors have shown that the use of a sandblaster is not required when the composition or paste of the present invention is used to manufacture the overlying opaque layer. The metal is then baked in a furnace at 980°C for about 9 minutes, where the metal is oxidised.

The dark colour of the metal implant can be masked by applying an opaque layer. During the opaque phase, the metal implant is painted with an opaque paste. The painted metal implant is then baked again in a furnace at 980°C for about 9 minutes. An optional second layer of opaque paint may then be applied.

Subsequently, the overlaying dental prosthesis can be manufactured. The composition in accordance with the invention is mixed with a dental powder in order to produce a dental paste as described elsewhere herein. During the modelling phase, the dental technician will carve and contour the paste into the desired shape to produce the dental prosthesis. The dental prosthesis will be processed, preferably using a micromotor. The processing step may involve polishing and/or trimming the dental prosthesis. In certain embodiments, the process comprises the step of polishing and/or trimming the dental prosthesis. Finally, the dental prosthesis is baked again in a furnace at 980°C for about 9 minutes, optionally with a dental glaze. The dental prosthesis may be baked at any suitable temperature for any suitable length of time to ensure that the dental paste cures and/or hardens. The skilled person is aware of suitable temperatures and times that may be used during the baking phase. In particular embodiments, the process comprises the step of baking the dental prosthesis at at least 980°C. In particular embodiments, the process comprises the step of baking the dental prosthesis at at least 900°C, at least 910°C, at least 920°C, at least 930°C, at least 940°C, at least 950°C, at least 960°C, at least 970°C, at least 980°C, at least 990°C, or at least 1000°C. In particular embodiments, the process comprises the step of baking the dental prosthesis in a furnace for at least 5 minutes, at least 6 minutes, at least 7 minutes, at least 8 minutes, at least 9 minutes, or at least 10 minutes. In particularly preferred embodiments, the process comprises the step of baking the dental prosthesis at at least 980°C for at least 9 minutes. A dental glaze may be applied to the external surface of the dental prosthesis to seal the open pores in the surface of a fired porcelain. Dental glazes may also produce a glossy surface. In certain embodiments, the process comprises the step of baking the dental prosthesis, optionally with a dental glaze.

In certain embodiments, the present invention provides a process for manufacturing a dental prosthesis, wherein the process comprises: a) a mixing phase; b) a modelling phase; c) a processing phase; d) a baking phase.

In certain embodiments, the invention provides a process for manufacturing a dental prosthesis, wherein the process comprises: a) mixing the composition in accordance with the invention with a dental powder to produce a dental paste; b) modelling the paste into a dental prosthesis; c) processing the dental prosthesis; and d) baking the dental prosthesis, optionally with a dental glaze.

All the embodiments described above in respect of the composition and the dental paste apply equally to this further aspect of the invention. In particular, the dental powder may be selected from: a ceramic powder, a porcelain powder, an opaque powder, an enamel powder, and a zirconium powder.

### F. Kits

For convenience, the materials used in the above described processes may be provided in kit form. These materials may be provided in a preformed dental paste of the present invention, or in conventional states. For example, the composition of the present invention and a dental powder may be provided separately. The kit may be provided with additional dental powders such as modelling powders, opaque powders or enamel powders, and other conventional materials as required. In certain embodiments, the present invention provides a kit comprising (i) the composition in accordance with the invention, (ii) a dental powder, and (iii) instructions for use. In certain embodiments, the present invention provides a kit comprising (i) the dental paste in accordance with the invention, and (ii) instructions for use.

### Incorporation bv Reference

Various publications are cited in the foregoing description and throughout the following examples, each of which is incorporated by reference herein in its entirety.

### EXAMPLES

The invention will be further understood with reference to the following non-limiting examples.

### Example 1: Production of a composition in accordance with the invention

### A. Trial and error: examples of unsuccessful formulas:

The inventors experimented with different quantities of distilled water, NaCl and KCI in order to identify a composition having the desired properties as described elsewhere herein. In an initial experiment, 150 ml of distilled water, 40 ml of NaCl (0.9%), and 60 ml of KCI (7.45%) were combined. For comparison, these quantities are shown as g/L, molar mass, and percentages in Table 1 below.

The composition was mixed with a dental powder in a weight ratio of 1:3 to produce a dental paste.

Upon modelling the paste it was immediately apparent that the water was evaporating too quickly from the paste. Drying occurred quickly and the consistency was unsatisfactory. This led to an undesired shape of the prosthesis.

The inventors also experimented with a composition having 180 ml of distilled water, 30 ml of NaCl (0.9%), and 40 ml of KCI (7.45%). For comparison, these quantities are shown as g/L, molar mass, and percentages in the table below. With these quantities, it was apparent that the composition was not suitable, and again the water evaporated too quickly from the paste.

### B. Trial and error: example of successful formula:

The inventors eventually found a formula for the composition that produced a suitable dental paste when combined with a dental powder. The formula combines 205 ml of distilled water, 25 ml of NaCl (0.9%), and 20 ml of KCI (7.45%). For comparison, these quantities are shown as g/L, molar mass, and percentages in the table below.

The composition was mixed with a dental powder in a weight ratio of 1:3 to produce a dental paste. The composition was tested with many different types of powders, including ceramic, porcelain, zirconium, enamel and opaque powders.

Upon modelling the paste, it was immediately observed that evaporation of the water took place slowly, which allowed the paste to be modelled on the bridge framework slowly. This gave time for the dental technician to craft the bridge layer by layer as the paste did not try up too quickly. Other advantages were apparent, for example, the dental paste comprising the composition formed a homogenous state and had an ideal viscosity that allowed for thin layers of the paste to be applied to the framework using a brush. It was found that the same advantages could be observed regardless of which type of powder was used. Thus, the composition of the present invention is compatible with a variety of types of dental powders, including opaque powders.

**Table 1: Calculation of salt quantities**

| | **NaCl** | **KCl** | **H20** |
|---|---|---|---|
| | 0.9% = 9 g/L | 7.45% = 74.5 g/L | |
| | | | |
| **Unsuccessful formula:** | (30 ml NaCl (0.9%)) - 0.27 g | (40 ml KCl (7.45%)) - 2.98 g | 180 ml |
| **In a total of 250 ml** | (30 ÷ 250) x 0.9 % | (40 ÷ 250) x 7.45 % | |
| **%** | **0.108 w/v%** | **1.192 w/v%** | 98.70 % |
| **g/L** | 1.08 q/L | 11.92 g/L | |
| **Molar** | 1.08 M | 11.92 M | |
| | | | |
| **Unsuccessful formula:** | (40 ml NaCl (0.9%) - 0.36 g | (60 ml KCI (7.45%) - 4.47 g | 150 ml |
| **In a total of 250 ml** | (40 ÷ 250) x 0.9 % | (60 ÷ 250) x 7.45 % | |
| **%** | **0.140 w/v%** | **1.788 w/v%** | 98.07 % |
| **g/L** | 1.44 g/L | 17.88 g/L | |
| **Molar** | 1.44 M | 17.88 M | |
| | | | |
| **Successful formula:** | (25 ml NaCl (0.9%) - 0.23 g | 20 ml KCl (7.45%) - 1.49 g | 205 ml |
| **In a total of 250 ml** | (25 ÷ 250) x 0.9 % | (20 ÷ 250) x 7.45 % | |
| **%** | **0.090 w/v%** | **0.596 w/v%** | 99.31 % |
| **g/L** | 0.9 g/L | 5.96 g/L | |
| **Molar** | 0.9 M | 5.96 M | |

Typically, the inventors found that an advantageous composition could be obtained by using up to about 35 ml of NaCl (0.9%) and up to about 35 ml of KCI (7.45%) in distilled water to a total volume of 250 ml (*i.e.* up to about 0.13 w/v% NaCl and up to about 1.04 w/v% KCI). Salt quantities beyond this did not yield satisfactory results.

### Example 2: Generation of a dental prosthesis

The composition and dental paste in accordance with the present invention have been used to produce a number of dental crowns and bridges. All remain stable with no repeated cracks/failures reported. The composition and dental paste in accordance with the present invention have also been used to successfully repair old and/or damaged crowns and bridges.

The composition in accordance with the invention was mixed with a dental powder in order to produce a dental paste as described elsewhere herein. Specifically, 0.09 w/v% NaCl and 0.60 w/v% KCI in water was mixed with a dental powder in a weight ratio of 1:3.

During the modelling phase, the dental technician carved and contoured the paste into the desired shape to produce the dental prosthesis. The dental prosthesis was processed using a micromotor to polish and trim the dental prosthesis. Finally, the dental prosthesis was baked in a furnace at 980°C for about 9 minutes with a dental glaze.

Figure 1 shows pictures of successful dental restoratives in accordance with the present invention.

## Claims

1. A composition for producing a dental paste, wherein the composition comprises:
(i) 0.01 to 0.13 w/v% NaCl and
(ii) 0.01 to 1.04 w/v% KCI
in water.

2. The composition according to claim 1, wherein the composition comprises:
(i) 0.05 to 0.13 w/v % NaCl and
(ii) 0.05 to 1.04 w/v % KCI
or
wherein the composition comprises:
(i) 0.08 to 0.13 w/v % NaCl and
(ii) 0.08 to 1.04 w/v % KCI
or
wherein the composition comprises:
(i) 0.1 w/v % NaCl and
(ii) 0.6 w/v % KCl
or
wherein the composition comprises:
(i) 0.09 w/v % NaCl and
(ii) 0.60 w/v % KCI

3. The composition according to claim 1 or claim 2, wherein the water is distilled water.

4. A dental paste comprising
(i) the composition according to any one of claims 1-3, and
(ii) a dental powder.

5. A dental paste according to claim 4,
wherein the ratio of composition to dental powder is 1:3; and/or
wherein the dental powder is selected from: a ceramic powder, a porcelain powder, an opaque powder, an enamel powder, a zirconium powder, and any combination thereof; optionally wherein the dental powder comprises silica (SiO₂), alumina (Al₂O₃), leucite, lithium disilicate, sodium aluminium silicate (soda feldspar), potassium aluminium silicate (potash feldspar), zirconia, and/or zirconium oxide; and optionally wherein the opaque powder comprises titanium oxide (TiO₂), aluminium oxide (Al₂O₃), and/or zirconium oxide (ZrO₂); and/or
wherein the composition forms 5-40 w/w% of the dental paste, preferably wherein the composition forms 10-20 w/w% of the dental paste.

6. The dental paste according to claim 4 or claim 5, further comprising a stabiliser and/or a preservative.

7. The dental paste according to any one of claims 4-6, wherein
(i) the dental paste is free of granules;
(ii) the dental paste is prevented from drying and cracking;
(iii) the colour, shade and/or luminescence of the dental paste is preserved during and after baking; and/or
(iv) the composition evaporates from the dental paste gradually.

8. Use of the composition according to any one of claims 1-3 or the dental paste according to any one of claims 4-7 for producing a dental prosthesis, optionally wherein the dental prosthesis is selected from: crowns, bridges, pontics, inlays, onlays, laminates, and veneers.

9. Use of the composition according to any one of claims 1-3 or the dental paste according to any one of claims 5-7 for producing an opaquer.

10. Use of the composition according to any one of claims 1-3 or the dental paste according to any one of claims 4-7 for
(i) patching enamel;
(ii) producing crowns;
(iii) producing bridges;
(iv) building veneers and laminates;
(v) preparing inlays and onlays;
(vi) producing a dental prosthesis;
(vii) preparing or correcting a dental restorative; or
(viii) connecting porcelain bridgework units.

11. A process for manufacturing a dental paste, wherein the process comprises mixing the composition according to any one of claims 1-3 with a dental powder.

12. A dental paste obtainable by the process of claim 11.

13. A process for manufacturing a dental prosthesis, wherein the process comprises:
a) mixing the composition according to any one of claims 1-3 with a dental powder to produce a dental paste;
b) modelling the paste into a dental prosthesis;
c) processing the dental prosthesis; and
d) baking the dental prosthesis, optionally with a dental glaze.

14. The process according to claim 11 or claim 13, wherein the dental powder is selected from: a ceramic powder, a porcelain powder, an opaque powder, an enamel powder, a zirconium powder, and any combination thereof.

15. A kit comprising:
(i) the composition according to any one of claims 1-3, and
(ii) a dental powder.
